# FASCICULE DE BREVET EUROPEEN

(11) **EP 2 079 881 B1**
(45) Date de publication et mention de la délivrance du brevet: **02.03.2011**
(21) Numéro de dépôt: 07858585.8
(22) Date de dépôt: 15.10.2007
(51) Int. Cl.: E02D 29/12

(54) **Dispositif de voirie à cadre de support et élément de couronnement monté articulé à basculement sur le cadre.**
Einstigsschacht mit Rahmen und daran schwenkbar angeordnetem Deckel.
Manhole frame with cover hinged thereto

(30) Priorité: 18.10.2006 FR 0654345
(43) Date de publication de la demande: 22.07.2009
(73) Titulaire: Norinco, 60149 St. Crepin Ibouvillers (FR)
(72) Inventeur: LACROIX, Pascal, 60600 Agnetz (FR)
(74) Mandataire: Thinat, Michel
(86) Numéro de dépôt international: PCT/FR2007/052158
(87) Numéro de publication internationale: WO 2008/050036

(56) Documents cités:
- EP-A1- 0 391 826
- DE-A1- 10 357 542
- DE-U1- 9 210 311
- FR-A1- 2 613 742
- FR-A1- 2 680 811
- GB-A- 341 180
- US-A- 5 282 339

## Description

La présente invention concerne un dispositif de voirie comprenant un cadre de support et un élément de couronnement ou de fermeture, tel qu'un tampon ou couvercle ou grille, monté articulé à basculement sur le cadre.

On connaît un tel dispositif selon lequel l'élément de couronnement est monté articulé à basculement sur le cadre le long d'une rive d'articulation par au moins une charnière d'articulation entre une position de fermeture du cadre dans laquelle l'élément de couronnement s'inscrit en épaisseur au moins en partie dans un rebord périphérique du cadre et est appliqué sur une feuillure du cadre, et une position d'ouverture renversée ou réciproquement.

Le document DE 103 5 7542 A divulgue un dispositif de voirie analogue à la préambule de la revendication 1.

Lorsque l'élément de couronnement occupe la position d'ouverture renversée sensiblement à 110° relativement au plan du bord d'ouverture du cadre, la charnière d'articulation reliant la rive de l'élément de couronnement au rebord correspondant du cadre est conçue de manière à assurer une sécurité empêchant l'élément de couronnement de basculer accidentellement à sa position d'obturation de l'ouverture du cadre.

Une telle articulation a pour inconvénient d'obliger un opérateur à soulever l'élément de couronnement relativement au cadre afin de désengager les moyens de la charnière d'articulation empêchant le basculement accidentel de l'élément de couronnement avant de faire basculer cet élément à sa position de fermeture du cadre. Cette opération préalable de soulèvement de l'élément de couronnement nécessite une certaine habileté de la part de l'opérateur pour débloquer la charnière d'articulation et exige de celui-ci un effort important lorsque l'élément de couronnement présente un poids élevé.

La présente invention a pour but d'éliminer l'inconvénient ci-dessus du dispositif de voirie connu.

A cet effet, selon l'invention, le dispositif de voirie comprenant un cadre de support et un élément de couronnement ou de fermeture, tel qu'un tampon ou couvercle ou grille, monté articulé à basculement sur le cadre le long d'une rive d'articulation par au moins une charnière d'articulation entre une position de fermeture du cadre dans laquelle l'élément de couronnement s'inscrit au moins en partie dans un rebord périphérique du cadre et est appliqué sur une feuillure du cadre et une position d'ouverture renversée ou réciproquement, est **caractérisé en ce qu'**il comprend un pêne solidaire de la face interne de l'élément de couronnement ou du rebord du cadre adjacent à la rive d'articulation de l'élément de couronnement et pouvant occuper une position active à laquelle une partie d'extrémité du pêne, lorsque l'élément de couronnement est basculé de sa position de fermeture à sa position d'ouverture, coopère avec une gâche du cadre ou de l'élément de couronnement pour retenir l'élément de couronnement à une position debout de sécurité empêchant le basculement de l'élément de couronnement à sa position de fermeture.

Le pêne, lorsque solidaire de l'élément de couronnement, s'étend transversalement à la rive d'articulation de l'élément de couronnement.

De préférence, en position active du pêne, sa partie d'extrémité fait saillie de la rive d'articulation de l'élément de couronnement et est en appui sur le bord d'une plaque formant gâche parallèle à la feuillure du cadre et solidaire perpendiculairement d'une partie du rebord du cadre adjacent à la rive d'articulation de l'élément de couronnement.

Le pêne peut être manoeuvré par un outil ou une clé de manière à le désengager de la gâche du cadre et permettre le libre basculement autour de la charnière d'articulation de l'élément de couronnement de sa position debout à sa position de fermeture.

Avantageusement, le pêne est monté à coulissement guidé dans deux glissières solidaires de la face interne de l'élément de couronnement perpendiculairement à la rive d'articulation de cet élément et est amené à sa position active en saillie de la rive d'articulation sous l'action d'un organe élastique, tel qu'un ressort hélicoïdal de compression.

En position de fermeture de l'élément de couronnement, le pêne est en appui par son extrémité libre et à l'encontre de la force de rappel de l'organe élastique sur la face interne du rebord du cadre adjacent à la rive d'articulation de l'élément de couronnement.

Le pêne s'étend parallèlement au voile plan de l'élément de couronnement.

Le pêne comprend un orifice auquel peut accéder l'outil ou la clé de manoeuvre au travers d'une l'ouverture formée dans le voile plan de l'élément de couronnement de manière à déplacer en translation le pêne, à l'encontre de la force de rappel de l'organe élastique, à sa position désengagée de la gâche du cadre lorsque l'élément de couronnement occupe sa position debout.

Avantageusement, l'orifice du pêne est un trou oblong traversant longitudinalement le pêne et l'ouverture du voile plan de l'élément de couronnement est un trou oblong parallèle et en regard du trou oblong du pêne.

L'extrémité du pêne opposée à sa partie active comporte une tête venant en appui sur une butée solidaire de la face interne de l'élément de couronnement lorsque l'organe élastique rappelle le pêne à sa position active de blocage de l'élément de couronnement à sa position debout.

La bande formant gâche s'étend le long du rebord correspondant du cadre et fait partie de la feuillure du cadre.

Dans le second mode de réalisation, le pêne, lorsque solidaire du rebord du cadre, s'étend transversalement à la direction longitudinale de ce rebord.

Le pêne peut être manoeuvré par un outil ou une clé de manière à le désengager de la gâche de l'élément de couronnement et permettre le libre basculement autour de la charnière d'articulation de l'élément de couronnement de sa position debout à sa position de fermeture.

Le pêne est monté à coulissement guidé dans un corps solidaire de la face interne du rebord du cadre et est amené à sa position active dans la gâche de l'élément de couronnement sous l'action d'un organe élastique, tel qu'un ressort hélicoïdal de compression.

En position de fermeture de l'élément de couronnement, le pêne est en appui par son extrémité libre et à l'encontre de la force de rappel de l'organe élastique sous la rive d'articulation de l'élément de couronnement.

Le pêne comprend deux ergots opposés transversaux à l'axe longitudinal du pêne et sur lesquels peut venir en appui une extrémité correspondante de l'outil de manoeuvre de manière à enfoncer le pêne dans le cadre, à l'encontre de la force de rappel de l'organe élastique, à sa position désengagée de la gâche de l'élément de couronnement lorsque ce dernier occupe sa position debout.

L'extrémité du pêne opposée à sa partie active comporte une tête venant en appui sur le corps de guidage du pêne solidaire de la face interne du rebord du cadre lorsque l'organe élastique rappelle le pêne à sa position active.

De préférence, le pêne peut être constitué par un barreau plat.

L'élément de couronnement peut occuper une position debout sensiblement égale à 90° relativement au cadre de support et sa position renversée sensiblement égale à 110° relativement à ce cadre.

L'invention sera mieux comprise, et d'autres buts, caractéristiques, détails et avantages de celle-ci apparaîtront plus clairement dans la description explicative qui va suivre faite en référence aux dessins schématiques annexés donnés uniquement à titre d'exemple illustrant un mode de réalisation de l'invention et dans lesquels :
- La figure 1 est une vue en perspective d'un dispositif de voirie à cadre de support et au moins un élément de couronnement monté articulé sur le cadre et occupant une position de fermeture de celui-ci ;
- la figure 2 est une vue partielle en perspective représentant l'un des éléments de couronnement de la figure 1 en position ouverte debout ;
- la figure 3 est une vue partielle en perspective suivant la flèche III de la figure 2 et représentant le pêne suivant un premier mode de réalisation de l'invention en position de blocage de l'élément de couronnement à sa position debout ;
- la figure 4 est une vue semblable à celle de la figure 3 et représentant le pêne à sa position de déblocage de l'élément de couronnement à sa position debout ;

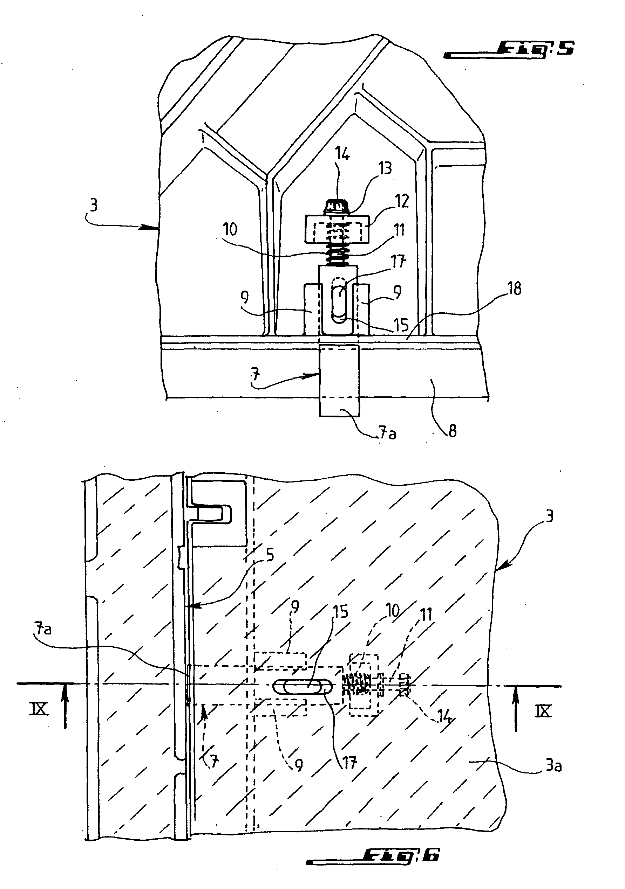

- la figure 5 est une vue suivant la flèche V de la figure 3 sans le cadre de support de l'élément de couronnement ;
- la figure 6 est une vue de dessus agrandie de la partie cerclée en VI de la figure 1 ;
- la figure 7 est une vue en perspective semblable à celle des figures 3 et 4 et représentant l'élément de couronnement en position totalement renversée relativement à son cadre de support ;
- la figure 8 est une vue en coupe agrandie suivant la ligne VIII-VIII de la figure 3 ;
- la figure 9 est une vue en coupe suivant la ligne IX-IX de la figure 6 ;
- la figure 10 est une vue partielle en coupe représentant un second mode de réalisation du pêne de l'invention à sa portion inactive à laquelle l'élément de couronnement est fermé ;
- la figure 11 représente l'élément de couronnement à deux positions ouvertes respectivement renversée et debout ; et
- la figure 12 est une vue de face suivant la flèche XII de la figure 11.

En se reportant aux figures, la référence 1 désigne un dispositif de voirie formant regard de chaussée réalisé en fonte et comprenant un cadre de support 2 et au moins un élément de couronnement ou de fermeture 3, tel qu'un tampon ou couvercle, ou une grille, monté articulé à basculement sur le cadre 2.

Un tel regard est disposé sur la voie publique pour accéder notamment à une cheminée de visite de galerie souterraine.

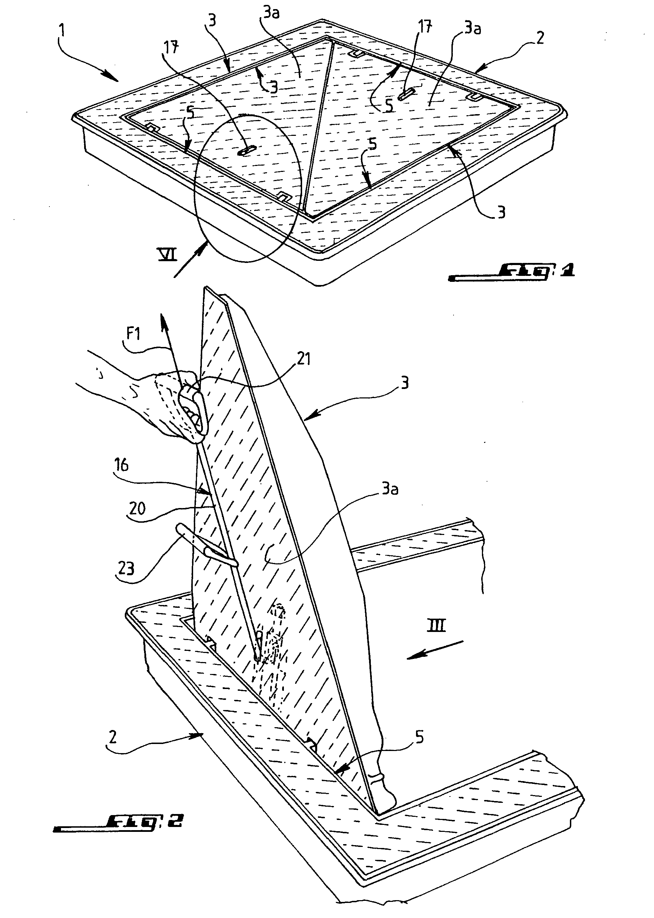

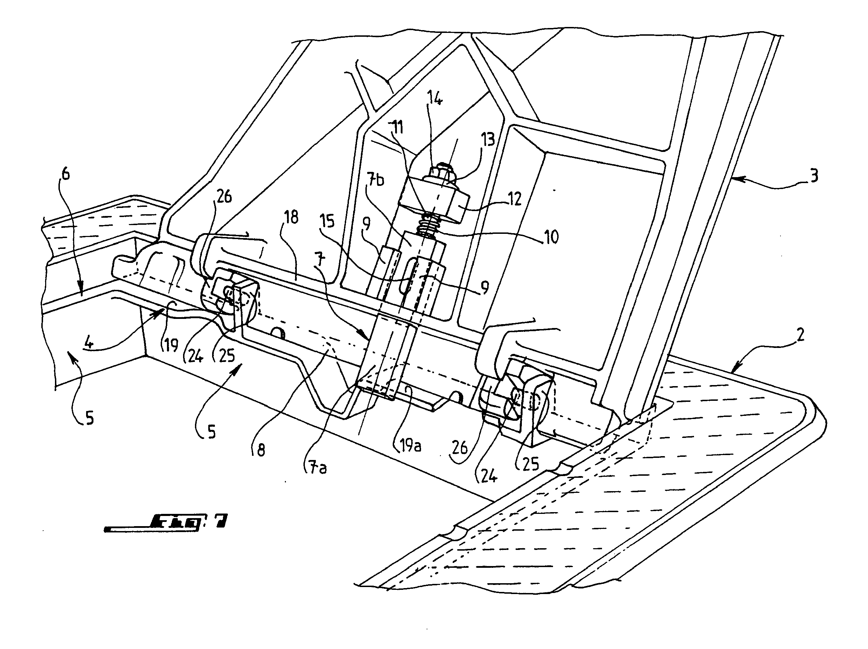

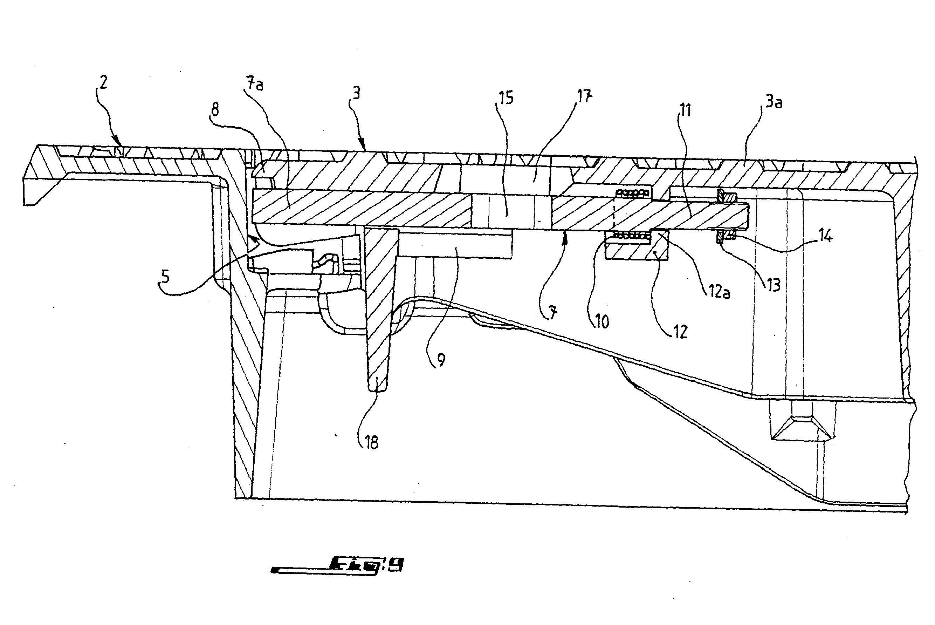

La figure 1 montre que le dispositif de voirie comprend deux éléments de couronnement 3 constitués par deux tampons ou plaques en forme de triangle rectangle montés articulés à basculement respectivement sur les deux côtés opposés du cadre 2 par l'intermédiaire de charnières d'articulation 4 de manière que chaque tampon 3 puisse basculer entre une position de fermeture du cadre 3 dans laquelle le tampon 3 s'inscrit en épaisseur en partie dans un rebord périphérique 5 du cadre 2 en étant appliqué sur une feuillure 6 de celui-ci comme représenté en figure 9 et une position d'ouverture renversée du cadre sensiblement à 110° relativement au plan du bord d'ouverture de ce cadre comme représenté en figure 7, ou réciproquement.

En position de fermeture des deux tampons en triangles rectangles 3, ceux-ci sont juxtaposés par leurs hypoténuses pour obturer complètement l'ouverture du cadre.

Bien entendu, l'élément de couronnement peut être constitué par un tampon de forme rectangulaire ou par une grille également de forme rectangulaire s'inscrivant dans un cadre conjugué.

Selon l'invention, chaque tampon 3 comprend, solidaire de sa face interne, un pêne 7 qui s'étend transversalement à la rive 8 du tampon 3 articulée par la charnière d'articulation 4 au rebord ou rive adjacent 5 du cadre 2.

Le pêne 7 est monté à coulissement guidé dans deux glissières parallèles 9 solidaires de la face interne du tampon 3 et s'étendant perpendiculairement à la rive d'articulation 8 du tampon 3.

Un ressort de compression 10 coopère avec le pêne 7 pour le déplacer en translation dans les deux glissières 9 dans un sens amenant une partie d'extrémité libre 7a de celui-ci à faire saillie au-delà de la rive d'articulation 8 lorsque le tampon 3 est basculé vers sa position d'ouverture renversée.

Lorsque le tampon 3 occupe sa position de fermeture du cadre 2, l'extrémité libre du pêne 7 est en appui sur une partie correspondante de la face interne du rebord vertical correspondant 5 du cadre 2 à l'encontre de la force de rappel du ressort 10.

Le pêne 7 est en forme générale de barreau plat métallique prolongé à son extrémité opposée à son extrémité 7a par une tige cylindrique 11 traversant une pièce formant butée 12 solidaire de la face interne du tampon 3. Le ressort 10 est monté coaxialement autour de la tige 11 entre l'extrémité 7b du pêne 7 et la pièce de butée 12. Cette dernière est creuse, présente une paroi d'extrémité 12a perpendiculaire au voile plan 3a du tampon 3 et traversée par la tige 11 du pêne 7, et sur la face interne de laquelle est en appui une extrémité du ressort 10. L'extrémité libre de la tige 11 du pêne 7 située à l'extérieur de la pièce de butée 12 comporte une rondelle 13 enfilée sur cette extrémité et retenue à celle-ci par l'intermédiaire, par exemple, d'un écrou 14 vissé à l'extrémité de la tige 11. Lorsque le pêne 7 occupe sa position active de blocage du tampon 3 au cadre 2 comme on le verra ultérieurement, la,rondelle 13 est en appui sur la paroi 12a de la pièce de butée 12 sous l'action de la force élastique exercée par le ressort 10 sur le pêne 7. La rondelle 13 peut être solidaire de l'écrou 14.

Le pêne 7 comporte un trou oblong traversant 15 s'étendant en direction longitudinale du pêne 7 et dans lequel peut être engagée l'extrémité d'un outil ou d'une clé 16 au travers d'un trou oblong 17 réalisé dans le voile plan 3a du tampon 3. Les deux trous oblongs 15, 17 sont parallèles l'un à l'autre et s'étendent symétriquement à un même plan perpendiculaire au voile 3a de manière à être en regard l'un de l'autre et permettre l'introduction de l'extrémité de l'outil 16 dans le trou oblong 15 du pêne 7 lorsque le tampon 3 occupe une position debout sensiblement perpendiculaire au cadre 2.

Le pêne 7 traverse une ouverture conjuguée réalisée dans une nervure 18 perpendiculaire à la face interne du tampon 3 et s'étendant sensiblement parallèlement à la rive d'articulation 8 du tampon.

La partie de feuillure 6 du cadre 2 au-dessus de laquelle est située la charnière d'articulation 4 est constituée par une bande 19 longeant la rive ou rebord correspondant 5 du cadre 2 et s'étendant perpendiculairement à ce rebord.

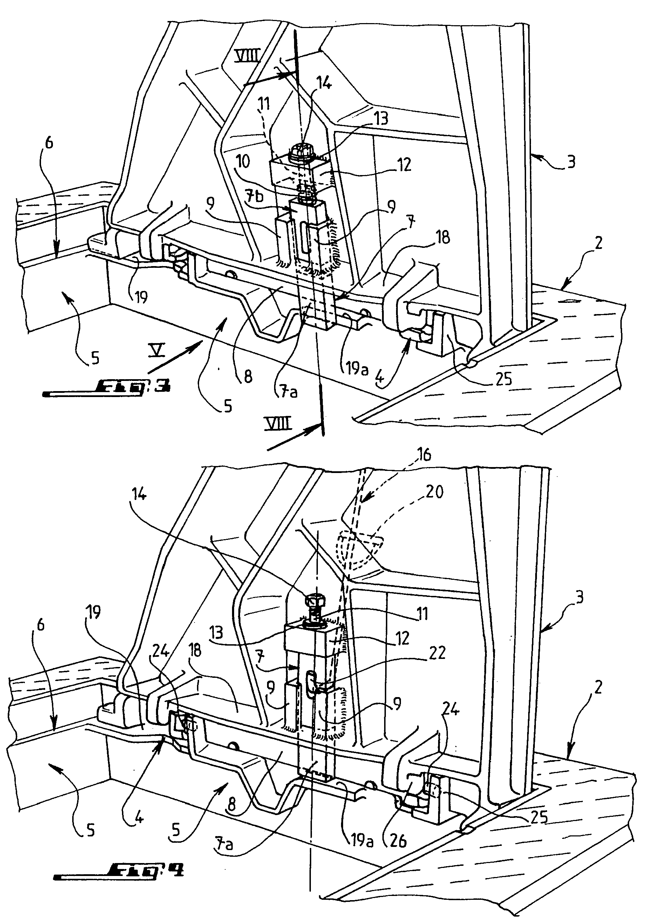

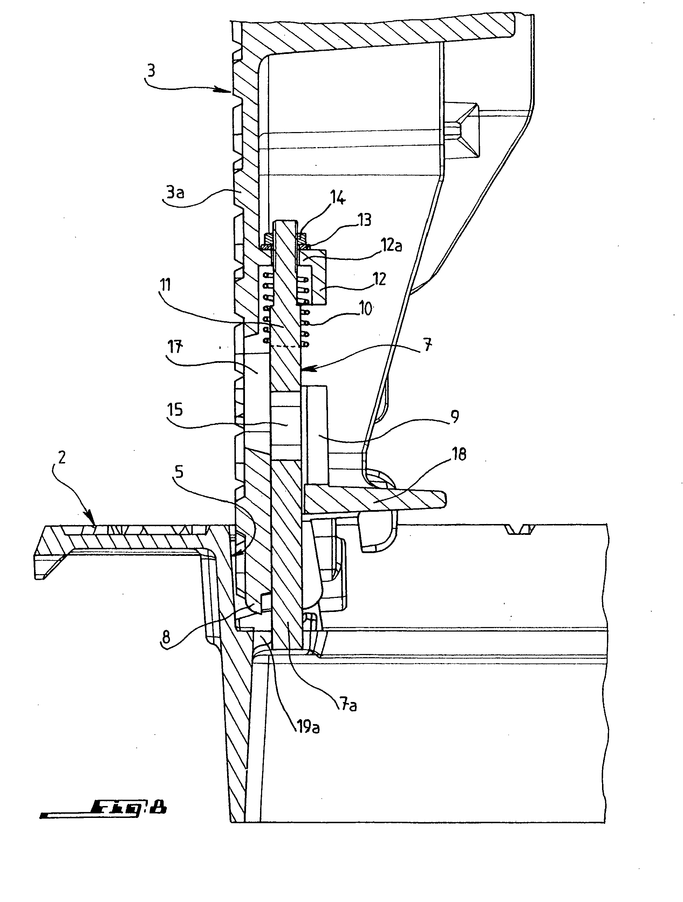

Comme cela ressort mieux des figures 3 et 8, en position debout sensiblement à 90° du tampon 3 relativement au cadre 2, la partie d'extrémité 7a du pêne 7 est en appui sur le bord d'une partie correspondante 19a de la bande 19 de manière à empêcher le basculement du tampon 3 de sa position debout à sa position d'obturation de l'ouverture du cadre 2.

La charnière d'articulation 4 du tampon 3 au cadre 2 est libre, c'est-à-dire que le tampon 3, en position inactive du pêne 7, peut librement basculer de sa position debout à sa position de fermeture du cadre 2.

L'outil 16 peut être constitué par une tige 20 ayant à l'une de ses extrémités une poignée de préhension 21 et son extrémité opposée en forme de crochet 22. Le cas échéant, l'outil 16 peut être pourvu d'une seconde poignée de manoeuvre 23 solidaire de la tige 20 entre la poignée 21 et l'extrémité recourbée 22 de l'outil 16.

L'utilisation du dispositif de voirie ressort déjà de la description qui en a été faite précédemment et va être maintenant expliquée.

Lorsque chaque tampon 3 occupe sa position horizontale de fermeture du cadre 2, la partie d'extrémité 7a du pêne 7 est en appui sous l'action du ressort 10 sur la partie correspondante du rebord vertical 5 du cadre 2 située au-dessus de la partie de bande 19a formant gâche.

Lorsque chaque tampon 3 est soulevé en pivotant autour de sa charnière d'articulation 4 au cadre 2, le ressort 10 déplace en translation le pêne 7 de manière que sa partie d'extrémité 7a fasse saillie au-delà de la rive d'articulation 8 du tampon 3 à la manière représentée en figure 7 et le tampon 3 peut être amené à sa position d'ouverture renversée de cette figure. Le pêne 7 est maintenu à cette position sortie par le ressort 10 et l'ensemble à rondelle 13 et écrou 14 en appui sur la pièce de butée 12.

Lorsque l'opérateur souhaite ramener chaque tampon 3 à sa position d'obturation du cadre 2, il fait basculer le tampon 3 autour de sa charnière d'articulation 4 au cadre 2 de sa position renversée de la figure 7 à sa position debout sensiblement perpendiculaire au cadre 2 de la figure 3. Le tampon 3 est maintenu à cette position debout par la partie d'extrémité 7a du pêne 7 en appui sur le bord correspondant de la bande 19a formant gâche de blocage du tampon 3 à cette position debout.

Ensuite, l'opérateur introduit l'extrémité recourbée 22 de l'outil 16 dans le trou oblong 17 du voile 3a du tampon 3 et le trou oblong 15 du pêne 7. L'opérateur peut alors exercer sur l'outil 16, à l'aide de la poignée 21 et, le cas échéant, de la poignée 23, un effort de traction au sens opposé au cadre 2 comme indiqué par la flèche F1 en figure 2 de manière à déplacer en translation vers le haut le pêne 7 jusqu'à ce que sa partie d'extrémité 7a soit située au-dessus de la bande formant gâche 19a du cadre 2. A cette position du pêne 7, le tampon 3 est déverrouillé du cadre 2 et peut librement basculer par gravité autour de sa charnière d'articulation 4 à sa position de fermeture du cadre 2.

Comme cela est connu en soi, le tampon 3 peut être maintenu relativement au cadre 2 à sa position ouverte et renversée d'environ 110° par deux ergots coaxiaux espacés 24 s'étendant suivant un axe parallèle au côté d'articulation correspondant du cadre 2 et solidaires respectivement de deux pattes 25 elles-mêmes solidaires de la bande formant feuillure 19 perpendiculairement à cette dernière. Chaque ergot 24 est logé dans un évidement d'une oreille correspondante 26 solidaire du tampon 3 et dont le fond vient en butée sur l'ergot 24 en position renversée du tampon 3 pour le bloquer à cette position.

Ainsi, le pêne 7 assure un moyen de sécurité coopérant avec la gâche 19a du cadre 2 pour empêcher le basculement accidentel du tampon 3 à sa position de fermeture. En outre, le basculement du tampon 3 de sa position debout à sa position de fermeture du cadre 2 est très simple puisqu'il suffit d'utiliser l'outil 16 pour déverrouiller le pêne 7 de sa gâche 19a et de faire basculer librement le tampon 3 autour de sa charnière d'articulation au cadre 2 sans avoir au préalable à soulever le tampon 3 relativement au cadre 2.

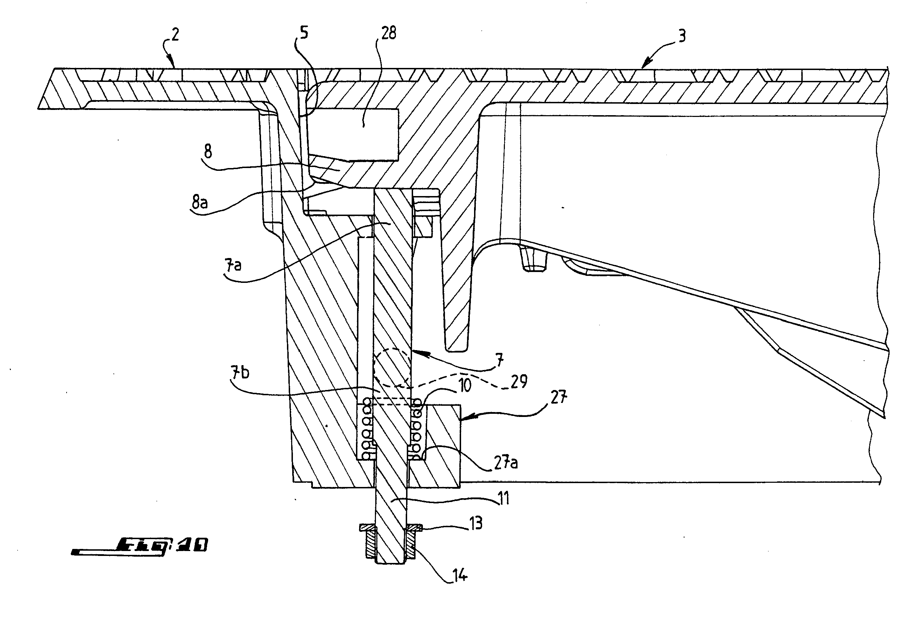

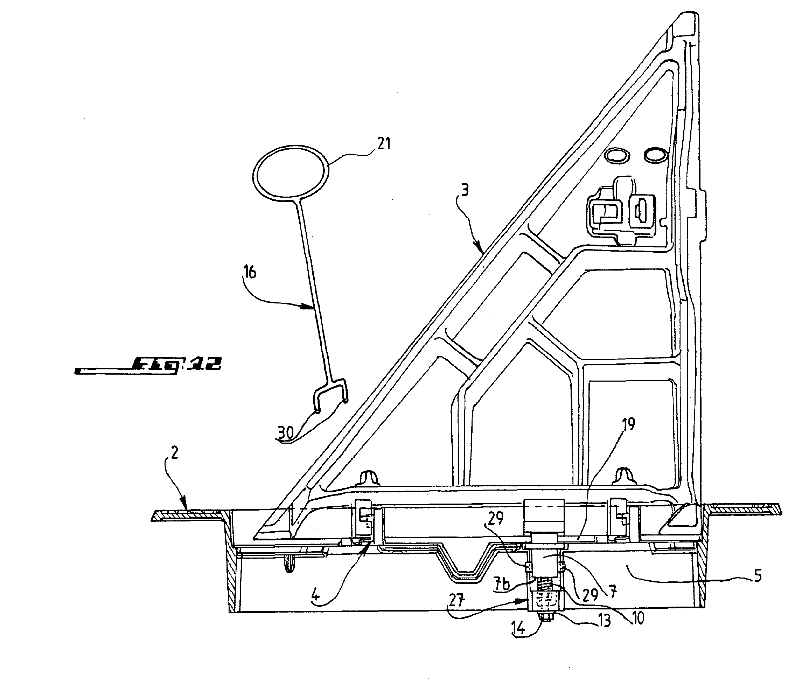

Les figures 10 à 12 représentent un second mode de réalisation selon lequel le pêne 7 est monté à coulissement guidé, suivant une direction perpendiculaire à la direction longitudinale du rebord 5 du cadre 2 adjacent à la rive d'articulation 8 de l'élément de couronnement 3, dans un corps 27 solidaire de la face interne de ce rebord.

Plus précisément, le pêne 7 a sa partie d'extrémité libre 7a traversant un orifice conjugué de la bande de feuillure 19 du rebord 5 et sa partie d'extrémité opposée 7b comportant une tige coaxiale 11 traversant le corps 27 qui peut venir de fonderie avec le cadre.

Comme représenté en figure 10, le pêne 7 occupe une position inactive à laquelle sa partie d'extrémité libre 7a est en appui sous la rive d'articulation 8 de l'élément de couronnement 3 à l'encontre de la force de rappel d'un organe élastique 10, tel que par exemple un ressort hélicoïdal de compression, monté coaxialement autour de la tige cylindrique 11 en étant en appui entre l'extrémité 7b du pêne 7 et le fond 27a du corps 27. Ainsi, l'ensemble constitué par le pêne 7 et le ressort 10 s'étend parallèlement à la face interne du rebord 5 du cadre 2.

L'extrémité libre de la tige 11 du pêne 7 située à l'extérieur du corps 27 comporte une rondelle 13 enfilée sur cette extrémité et retenue à celle-ci, par exemple, un écrou 14 vissé à l'extrémité de la tige 11. La rondelle 13 peut être solidaire de l'écrou 14. Lorsque le pêne 7 occupe sa position active de blocage du tampon 3 au cadre 2, l'ensemble à rondelle 13 et écrou 14 est en appui sur le corps 27 sous l'action de la force élastique exercée par le ressort 10 sur le pêne 7.

La rive d'articulation 8 du tampon 3 comporte approximativement dans sa partie médiane une ouverture 28 s'étendant perpendiculairement à la rive 8 et constituant une gâche de réception de la partie d'extrémité libre 7a du pêne 7 lorsque le tampon 3 occupe sa position d'ouverture.

La rive d'articulation 8 du tampon 3 comporte un bord 8a conformé en rampe ou en came permettant de guider la partie d'extrémité libre 7a du pêne 7 dans la gâche 28 lors du basculement du tampon 3 à sa position d'ouverture.

Le pêne 7 comprend deux ergots opposés 29 transversaux à l'axe longitudinal du pêne 7 en étant disposés symétriquement relativement au plan longitudinal de ce pêne et qui peuvent coopérer avec une extrémité en forme de fourche à deux doigts 30 d'un outil de manoeuvre 16 de manière à enfoncer le pêne 7, à l'encontre de la force de rappel du ressort 10, dans le cadre 2 pour désengager la partie d'extrémité 7a du pêne 7 de la gâche 28 en position debout du tampon 3.

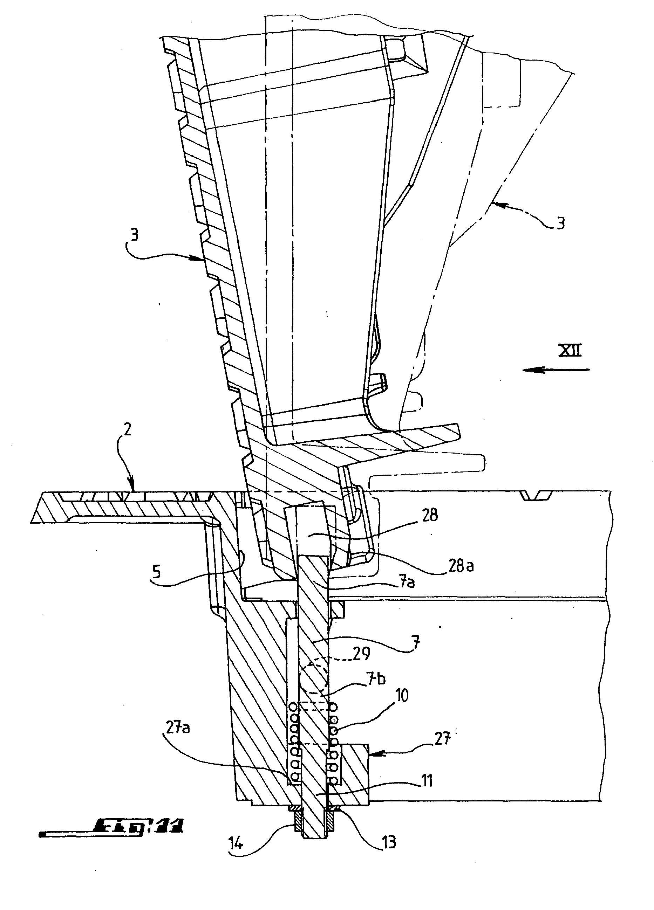

Lorsque chaque tampon 3 est soulevé à partir de sa position de fermeture de la figure 10, il pivote autour de sa charnière d'articulation 4 et le bord interne en came 8a de la rive d'articulation 8 du tampon 3 se déplace sur la partie d'extrémité libre 7a du pêne 7 jusqu'à ce que celle-ci s'engage automatiquement, sous l'action de la force de rappel exercée par le ressort 10, dans l'ouverture formant gâche 28 lorsque le tampon 3 est basculé à sa position renversée représentée en figure 11. A cette position, le tampon 3 est maintenu par coopération des ergots 24 et oreilles 26 comme dans le premier mode de réalisation. Lorsque le tampon 3 est basculé accidentellement de sa position renversée à sa position debout sensiblement à 90° relativement au cadre 2 comme représenté en traits mixtes en figure 11, la partie d'extrémité libre 7a du pêne 7 engagée dans la gâche 8 coopère avec cette dernière pour retenir le tampon 3 à cette position debout et l'empêcher par conséquent de basculer à sa position de fermeture du cadre 2.

Pour fermer à nouveau l'ouverture du cadre 2 par le tampon 3, un opérateur utilise l'outil 16 de manière que les deux doigts en fourche 30 viennent en appui respectivement sur les deux ergots 29 du pêne 7 pour enfoncer ce dernier dans le cadre 2 à l'encontre de la force de rappel du ressort 10 et l'opérateur peut alors faire basculer manuellement et librement autour de sa charnière d'articulation 4 le tampon 3 à sa position de fermeture de la figure 10.

## Revendications

1. Dispositif de voirie (1) comprenant un cadre de support (2) et un élément de couronnement ou de fermeture (3), tel qu'un tampon ou couvercle ou grille, monté articulé à basculement sur le cadre (2) le long d'une rive d'articulation (8) par au moins une charnière d'articulation (4) entre une position de fermeture du cadre (2) dans laquelle l'élément de couronnement (3) s'inscrit au moins en partie dans un rebord périphérique (5) du cadre (2) et est appliqué sur une feuillure (6) du cadre (2) et une position d'ouverture renversée ou réciproquement, **caractérisé en ce qu'**il comprend un pêne, solidaire de la face interne de l'élément de couronnement ou du rebord du cadre adjacent à la rive d'articulation (8) de l'élément de couronnement (3) et pouvant occuper une position active à laquelle une partie d'extrémité (7a) du pêne (7), lorsque l'élément de couronnement (3) est basculé de sa position de fermeture à sa position d'ouverture, coopère avec une gâche (19a, 28) du cadre (2) ou de l'élément de couronnement pour retenir l'élément de couronnement (3) à une position debout de sécurité empêchant le basculement de l'élément de couronnement (3) à sa position de fermeture.

2. Dispositif selon la revendication 1, **caractérisé en ce que** le pêne, lorsque solidaire de l'élément de couronnement, s'étend transversalement à la rive d'articulation (8) de l'élément de couronnement.

3. Dispositif selon la revendication 1, **caractérisé en ce que**, en position active du pêne (7), sa partie d'extrémité (7a) fait saillie de la rive d'articulation (8) de l'élément de couronnement (3) et la partie en saillie (7a) du pêne (7) est en appui sur le bord d'une plaque (19a) formant gâche parallèle à la feuillure (6) du cadre (2) et solidaire perpendiculairement d'une partie du rebord (5) du cadre (2) adjacent à la rive d'articulation (8) de l'élément de couronnement (3).

4. Dispositif selon la revendication 1 ou 2, **caractérisé en ce que** le pêne (7) peut être manoeuvré par un outil ou une clé (16) de manière à le désengager de la gâche (19a) du cadre (2) et permettre le libre basculement autour de la charnière d'articulation (4) de l'élément de couronnement (3) de sa position debout à sa position de fermeture.

5. Dispositif selon l'une des revendications précédentes, **caractérisé en ce que** le pêne (7) est monté à coulissement guidé dans deux glissières (9) solidaires de la face interne de l'élément de couronnement (3) perpendiculairement à la rive d'articulation (8) de cet élément et est amené à sa position active en saillie de la rive d'articulation (8) sous l'action d'un organe élastique (10), tel qu'un ressort hélicoïdal de compression.

6. Dispositif selon la revendication 5, **caractérisé en ce qu'**en position de fermeture de l'élément de couronnement (3), le pêne (7) est en appui par son extrémité libre (7a) et à l'encontre de la force de rappel de l'organe élastique (10) sur la face interne du rebord (5) du cadre (2) adjacent à la rive d'articulation (8) de l'élément de couronnement (3).

7. Dispositif selon l'une des revendications précédentes, **caractérisé en ce que** le pêne (7) s'étend parallèlement au voile plan (3a) de l'élément de couronnement (3).

8. Dispositif selon l'une des revendications 4 à 6, **caractérisé en ce que** le pêne (7) comprend un orifice (15) auquel peut accéder l'outil ou la clé de manoeuvre (16) au travers d'une ouverture (17) formée dans le voile plan (3a) de l'élément de couronnement (3) de manière à déplacer en translation le pêne (7), à l'encontre de la force de rappel de l'organe élastique (10), à sa position désengagée de la gâche (19a) du cadre (2) lorsque l'élément de couronnement (3) occupe sa position debout.

9. Dispositif selon la revendication 7, **caractérisé en ce que** l'orifice du pêne (7) est un trou oblong (15) traversant longitudinalement le pêne (7) et l'ouverture du voile plan (3a) de l'élément de couronnement (3) est un trou oblong (17) parallèle et en regard du trou oblong (15) du pêne (7).

10. Dispositif selon l'une des revendications 4 à 8, **caractérisé en ce que** l'extrémité (7b) du pêne (7) opposée à sa partie active (7a) comporte une tête (13,14) venant en appui sur une butée (12) solidaire de la face interne de l'élément de couronnement (3) lorsque l'organe élastique (10) rappelle le pêne (7) à sa position active de blocage de l'élément de couronnement (3) à sa position debout.

11. Dispositif selon l'une des revendications 2 à 10, **caractérisé en ce que** la bande formant gâche (19a) s'étend le long du rebord correspondant (5) du cadre (2) et fait partie de la feuillure (6) du cadre (2).

12. Dispositif selon la revendication 1, **caractérisé en ce que** le pêne (7), lorsque solidaire du rebord du cadre (2), s'étend transversalement à la direction longitudinale de ce rebord.

13. Dispositif selon la revendication 12, **caractérisé en ce que** le pêne (7) peut être manoeuvré par un outil ou une clé (16) de manière à le désengager de la gâche de l'élément de couronnement (3) et permettre le libre basculement autour de la charnière d'articulation (4) de l'élément de couronnement (3) de sa position debout à sa position de fermeture.

14. Dispositif selon la revendication 12 ou 13, **caractérisé en ce que** le pêne (7) est monté à coulissement guidé dans un corps (27) solidaire de la face interne du rebord du cadre (3) et est amené à sa position active dans la gâche (28) de l'élément de couronnement (3) sous l'action d'un organe élastique (10), tel qu'un ressort hélicoïdal de compression.

15. Dispositif selon l'une des revendications 12 à 14, **caractérisé en ce qu'**en position de fermeture de l'élément de couronnement (3), le pêne (7) est en appui par son extrémité libre (7a) et à l'encontre de la force de rappel de l'organe élastique (10) sous la rive d'articulation (8) de l'élément de couronnement (3).

16. Dispositif selon l'une des revendications 12 à 15, **caractérisé en ce que** le pêne (7) comprend deux ergots opposés (29) transversaux à l'axe longitudinal du pêne (7) et sur lesquels peut venir en appui une extrémité correspondante de l'outil de manoeuvre (16) de manière à enfoncer le pêne (7) dans le cadre (2), à l'encontre de la force de rappel de l'organe élastique (10), à sa position désengagée de la gâche (28) de l'élément de couronnement (3) lorsque ce dernier occupe sa position debout.

17. Dispositif selon l'une des revendications 12 à 16, **caractérisé en ce que** l'extrémité (7b) du pêne (7) opposée à sa partie active (7a) comporte une tête (13, 14) venant en appui sur le corps de guidage(27) du pêne (7) solidaire de la face interne du rebord du cadre (2) lorsque l'organe élastique (10) rappelle le pêne (7) à sa position active.

18. Dispositif selon l'une des revendications précédentes, **caractérisé en ce que** le pêne (7) est constitué par un barreau plat.

19. Dispositif selon l'une des revendications précédentes, **caractérisé en ce que** l'élément de couronnement (3) peut occuper une position debout sensiblement égale à 90° relativement au cadre de support (2) et sa position renversée sensiblement égale à 110° relativement à ce cadre.

## Claims

1. A road system device (1), comprising a support frame (2) and a cover or closing element (3), such as a plug or lid or grid, hingedly mounted so as to tilt on the frame (2) along a hinge shoulder (8) of the cover element (3) by at least one articulated hinge (4) between a closing position of the frame (2), in which the cover element (3) fits at least partially into a peripheral rim (5) of the frame (2) and is applied to a rebate (6) of the frame (2), and an inverted opening position, or vice versa, **characterized in that** it comprises a lock bolt, integral with the internal surface of the cover element or the rim of the frame adjacent to the hinge shoulder (8) of the cover element (3) and capable of occupying an active position, in which an end part (7a) of the lock bolt (7), when the cover element (3) is tilted from the closing position thereof to the opening position thereof, cooperates with a keeper (19a, 28) of the frame (2) or the cover element so as to retain the cover element (3) in an upright safety position preventing the cover element (3) from tilting towards the closing position thereof.

2. The device according to claim 1, **characterized in that** the lock bolt, when integral with the cover element, extends transversely to the hinge shoulder (8) of the cover element.

3. The device according to claim 1, **characterized in that** in the active position of the lock bolt (7), the end part (7a) thereof protrudes from the hinge shoulder (8) of the cover element (3), and the protruding part (7a) of the lock bolt (7) is resting on the edge of a keeper plate (19a) parallel to the rebate (6) of the frame (2) and perpendicularly integral with one part of the rim (5) of the frame (2) adjacent to the hinge shoulder (8) of the cover element (3).

4. The device according to claim 1 or 2, **characterized in that** the lock bolt (7) can be manipulated by a tool or a key (16) so as to disengage the same from the keeper (19a) of the frame (2) and allow for free tilting around the articulated hinge (4) of the cover element (3) from the upright position thereof to the closing position thereof.

5. The device according to any of the preceding claims, **characterized in that** the lock bolt (7) is slidingly mounted while being guided in two rails (9) integral with the internal surface of the cover element (3) perpendicularly to the hinge shoulder (8) of this element, and is brought into the active position thereof, protruding from the hinge shoulder (8) under the effect of an elastic member (10), such as a coil spring.

6. The device according to claim 5, **characterized in that** in the closing position of the cover element (3), the lock bolt (7) is resting with the free end (7a) thereof and against the return force of the elastic member (10) on the internal surface of the rim (5) of the frame (2) adjacent to the hinge shoulder (8) of the cover element (3).

7. The device according to any of the preceding claims, **characterized in that** the lock bolt (7) extends in parallel to the flat cover plate (3a) of the cover element (3).

8. The device according to any of claims 4 to 6, **characterized in that** the lock bolt (7) comprises an orifice (15) which the manipulating tool or key (16) can access through an opening (17) formed in the flat cover plate (3a) of the cover element (3) so as to translate the lock bolt (7), against the return force of the elastic member (10), to the position thereof disengaged from the keeper (19a) of the frame (2) when the cover element (3) is in the upright position thereof.

9. The device according to claim 7, **characterized in that** the orifice of the lock bolt (7) is an elongated hole (15) passing lengthwise through the lock bolt (7), and the opening of the flat cover plate (3a) of the cover element (3) is an elongated hole (17) parallel and opposite the elongated hole (15) of the lock bolt (7).

10. The device according to any of claims 4 to 8, **characterized in that** the end (7b) of the lock bolt (7) opposite the active part (7a) thereof comprises a head (13, 14) resting on a stop (12) integral with the internal surface of the cover element (3) when the elastic member (10) returns the lock bolt (7) to the active position thereof blocking the cover element (3) in the upright position thereof.

11. The device according to any of claims 2 to 10, **characterized in that** the keeper band (19a) extends along the corresponding rim (5) of the frame (2) and is part of the rebate (6) of the frame (2).

12. The device according to claim 1, **characterized in that** the lock bolt (7), when integral with the rim of the frame (2), extends transversely to the longitudinal direction of the rim.

13. The device according to claim 12, **characterized in that** the lock bolt (7) can be manipulated by a tool or a key (16) so as to disengage the same from the keeper of the cover element (3) and allow for free tilting around the articulated hinge (4) of the cover element (3) from the upright position thereof to the closing position thereof.

14. The device according to claim 12 or 13, **characterized in that** the lock bolt (7) is slidingly mounted while being guided in a body (27) integral with the internal surface of the rim of the frame (3), and is brought into the active position thereof inside the keeper (28) of the cover element (3) under the effect of an elastic member (10), such as a coil spring.

15. The device according to any of claims 12 to 14, **characterized in that** in the closing position of the cover element (3), the lock bolt (7) is resting with the free end (7a) thereof and against the return force of the elastic member (10) under the hinge shoulder (8) of the cover element (3).

16. The device according to any of claims 12 to 15, **characterized in that** the lock bolt (7) comprises two opposite stubs (29) transverse to the longitudinal axis of the lock bolt (7) and on which a corresponding end of the manipulating tool (16) can come to rest so as to force the lock bolt (7) into the frame (2), against the return force of the elastic member (10), into the position thereof disengaged from the keeper (28) of the cover element (3) when the latter is in the upright position thereof.

17. The device according to any of claims 12 to 16, **characterized in that** the end (7b) of the lock bolt (7) opposite the active part (7a) thereof comprises a head (13, 14) coming to rest on the guiding body (27) of the lock bolt (7) integral with the internal surface of the rim of the frame (2) when the elastic member (10) returns the lock bolt (7) to the active position thereof.

18. The device according to any of the preceding claims, **characterized in that** the lock bolt (7) is made of a flat bar.

19. The device according to any of the preceding claims, **characterized in that** the cover element (3) can occupy an upright position substantially equal to 90° in relation to the support frame (2) and the inverted position thereof substantially equal to 110° in relation to the frame.

## Patentansprüche

1. Straßenvorrichtung (1) umfassend einen Tragrahmen (2) und ein Aufsatz- oder Schließelement (3), wie etwa eine Abdeckung oder einen Deckel oder ein Gitter, das an dem Rahmen (2) an einem Gelenkrandanschlag (8) entlang über mindestens ein Gelenkscharnier (4) zwischen einer Verschlussposition des Rahmens (2), in der das Aufsatzelement (3) mindestens teilweise in eine umlaufende Einfassung (5) des Rahmens (2) passt und an einen Anschlag (6) des Rahmens (2) angelegt ist, und einer umgekehrten Öffnungsposition, oder umgekehrt kippbar gelenkig montiert ist, **dadurch gekennzeichnet, dass** sie einen Sperrbolzen umfasst, der mit der Innenseite des Aufsatzelements oder der Einfassung des Rahmens neben dem Gelenkrandanschlag (8) des Aufsatzelements (3) fest verbunden ist und eine aktive Position einnehmen kann, in der ein Endteil (7a) des Sperrbolzens (7), wenn das Aufsatzelement (3) von seiner Verschlussposition in seine Öffnungsposition gekippt wird, mit einer Raste (19a, 28) des Rahmens (2) oder des Aufsatzelements zusammenwirkt, um das Aufsatzelement (3) in einer aufrechten Sicherheitsposition festzuhalten, die das Kippen des Aufsatzelements (3) in seine Verschlussposition verhindert.

2. Vorrichtung nach Anspruch 1, **dadurch gekennzeichnet, dass** der Sperrbolzen, wenn er mit dem Aufsatzelement fest verbunden ist, sich quer zum Gelenkrandanschlag (8) des Aufsatzelements erstreckt.

3. Vorrichtung nach Anspruch 1, **dadurch gekennzeichnet, dass** in der aktiven Position des Sperrbolzens (7) ein Endteil (7a) über den Gelenkrandanschlag (8) des Aufsatzelements (3) übersteht und der überstehende Teil (7a) des Sperrbolzens (7) auf dem Rand einer Rastenplatte (19a) aufliegt, die parallel zum Anschlag (6) des Rahmens (2) ist und mit einem Teil der Einfassung (5) des Rahmens (2) neben dem Gelenkrandanschlag (8) des Aufsatzelements (3) rechtwinklig fest verbunden ist.

4. Vorrichtung nach Anspruch 1 oder 2, **dadurch gekennzeichnet, dass** der Sperrbolzen (7) mit einem Werkzeug oder einem Schlüssel (16) betätigt werden kann, um ihn aus der Raste (19a) des Rahmens (2) zu lösen und das freie Kippen um das Gelenkscharnier (4) des Aufsatzelements (3) von seiner aufrechten Position in seine Verschlussposition zu ermöglichen.

5. Vorrichtung nach einem der vorhergehenden Ansprüche, **dadurch gekennzeichnet, dass** der Sperrbolzen (7) in zwei Schienen (9) geführt gleitend montiert ist, die mit der Innenseite des Aufsatzelements (3) rechtwinklig zum Gelenkrandanschlag (8) dieses Elements fest verbunden sind, und in seine über den Gelenkrandanschlag (8) vorstehende aktive Position unter der Einwirkung eines elastischen Organs (10), wie etwa einer Spiraldruckfeder, gebracht wird.

6. Vorrichtung nach Anspruch 5, **dadurch gekennzeichnet, dass** in der Verschlussposition des Aufsatzelements (3) der Sperrbolzen (7) mit seinem freien Ende (7a) und gegen die Rückstellkraft des elastischen Organs (10) auf der Innenseite der Einfassung (5) des Rahmens (2) neben dem Gelenkrandanschlag (8) des Aufsatzelements (3) anliegt.

7. Vorrichtung nach einem der vorhergehenden Ansprüche, **dadurch gekennzeichnet, dass** der Sperrbolzen (7) sich parallel zu der ebenen Abdeckplatte (3a) des Aufsatzelements (3) erstreckt.

8. Vorrichtung nach einem der Ansprüche 4 bis 6, **dadurch gekennzeichnet, dass** der Sperrbolzen (7) eine Mündung (15) umfasst, auf die das Betätigungswerkzeug oder der Betätigungsschlüssel (16) durch eine Öffnung (17), die in der ebenen Abdeckplatte (3a) des Aufsatzelements (3) gebildet ist, zugreifen kann, so dass der Sperrbolzen (7) gegen die Rückstellkraft des elastischen Organs (10) in seine aus der Raste (19a) des Rahmens (2) gelösten Position in einer Translationsbewegung verschoben wird, wenn das Aufsatzelement (3) seine aufrechte Position einnimmt.

9. Vorrichtung nach Anspruch 7, **dadurch gekennzeichnet, dass** die Mündung des Sperrbolzens (7) ein längliches Loch (15) ist, das längsseitig den Sperrbolzen (7) durchquert, und die Öffnung der ebenen Abdeckplatte (3a) des Aufsatzelements (3) ein längliches Loch (17) ist, das zu dem länglichen Loch (15) des Sperrbolzens (7) parallel ist und diesem gegenüberliegt.

10. Vorrichtung nach einem der Ansprüche 4 bis 8, **dadurch gekennzeichnet, dass** das Ende (7b) des Sperrbolzens (7), das seinem aktiven Teil (7a) gegenüberliegt, einen Kopf (13, 14) umfasst, der an einem Anschlag (12) anliegt, der mit der Innenseite des Aufsatzelements (3) fest verbunden ist, wenn das elastische Organ (10) den Sperrbolzen (7) in seine aktive Position zum Blockieren des Aufsatzelements (3) in seine aufrechte Position zurückstellt.

11. Vorrichtung nach einem der Ansprüche 2 bis 10, **dadurch gekennzeichnet, dass** das Rastenband (19a) sich an der entsprechenden Einfassung (5) des Rahmens (2) entlang erstreckt und Teil des Anschlags (6) des Rahmens (2) ist.

12. Vorrichtung nach Anspruch 1, **dadurch gekennzeichnet, dass** der Sperrbolzen (7), wenn er mit der Einfassung des Rahmens (2) fest verbunden ist, sich quer zur Längsrichtung dieser Einfassung erstreckt.

13. Vorrichtung nach Anspruch 12, **dadurch gekennzeichnet, dass** der Sperrbolzen (7) von einem Werkzeug oder einem Schlüssel (16) derart betätigt werden kann, dass er aus der Raste des Aufsatzelements (3) gelöst wird und das freie Kippen um das Gelenkscharnier (4) des Aufsatzelements (3) von seiner aufrechten Position in seine Verschlussposition ermöglicht wird.

14. Vorrichtung nach Anspruch 12 oder 13, **dadurch gekennzeichnet, dass** der Sperrbolzen (7) in einem Körper (27) gleitend geführt montiert ist, der mit der Innenseite der Einfassung des Rahmens (3) fest verbunden ist, und in seine aktive Position in der Raste (28) des Aufsatzelements (3) unter der Einwirkung eines elastischen Organs (10), wie etwa einer Spiraldruckfeder, gebracht wird.

15. Vorrichtung nach einem der Ansprüche 12 bis 14, **dadurch gekennzeichnet, dass** in der Verschlussposition des Aufsatzelements (3) der Sperrbolzen (7) mit seinem freien Ende (7a) und gegen die Rückstellkraft des elastischen Organs (10) unter dem Gelenkrandanschlag (8) des Aufsatzelements (3) anliegt.

16. Vorrichtung nach einem der Ansprüche 12 bis 15, **dadurch gekennzeichnet, dass** der Sperrbolzen (7) zwei gegenüberliegende Ansätze (29) quer zur Längsachse des Sperrbolzens (7) umfasst, und an denen ein entsprechendes Ende des Betätigungswerkzeugs (16) anliegen kann, um den Sperrbolzen (7) gegen die Rückstellkraft des elastischen Organs (10) in seine aus der Raste (28) des Aufsatzelements (3) gelöste Position in den Rahmen (2) zu drücken, wenn das Aufsatzelement seine aufrechte Position einnimmt.

17. Vorrichtung nach einem der Ansprüche 12 bis 16, **dadurch gekennzeichnet, dass** das Ende (7b) des Sperrbolzens (7) gegenüber seinem aktiven Teil (7a) einen Kopf (13, 14) umfasst, der an dem Führungskörper (27) des Sperrbolzens (7) anliegt, der mit der Innenseite der Einfassung des Rahmens (2) fest verbunden ist, wenn das elastische Organ (10) den Sperrbolzen (7) in seine aktive Position zurückstellt.

18. Vorrichtung nach einem der vorhergehenden Ansprüche, **dadurch gekennzeichnet, dass** der Sperrbolzen (7) aus einer flachen Stange besteht.

19. Vorrichtung nach einem der vorhergehenden Ansprüche, **dadurch gekennzeichnet, dass** das Aufsatzelement (3) eine aufrechte Position, die im Wesentlichen gleich 90° zum Tragrahmen (2) ist, und seine umgekehrte Position, die im Wesentlichen gleich 110° zu diesem Rahmen ist, einnehmen kann.
